(19) Europäisches Patentamt / European Patent Office / Office européen des brevets

(11) **EP 3 801 655 B1**

(12) **EUROPEAN PATENT SPECIFICATION**

(45) Date of publication and mention
of the grant of the patent:
**10.05.2023   Bulletin 2023/19**

(21) Application number: **20711290.5**

(22) Date of filing: **10.03.2020**

(51) International Patent Classification (IPC):
**A61L 24/00** *(2006.01)*        **C09J 183/00** *(2006.01)*

(52) Cooperative Patent Classification (CPC):
(C-Sets available)
**A61L 24/0031; A61L 24/001; A61L 24/0036;
A61L 24/0094; C09J 183/04;** C08G 77/12;
C08G 77/20                                    (Cont.)

(86) International application number:
**PCT/GB2020/050563**

(87) International publication number:
**WO 2020/201688 (08.10.2020 Gazette 2020/41)**

(54) **FOAMED SKIN COMPATIBLE SILICONE COMPOSITION**

GESCHÄUMTE HAUTVERTRÄGLICHE SILIKONZUSAMMENSETZUNG

COMPOSITION DE SILICONE EXPANSÉE COMPATIBLE AVEC LA PEAU

(84) Designated Contracting States:
**AL AT BE BG CH CY CZ DE DK EE ES FI FR GR
HR HU IE IS IT LI LT LU LV MC MK MT NL NO PL
PT RO RS SE SI SK SM TR**

(30) Priority: **29.03.2019   GB 201904402**

(43) Date of publication of application:
**14.04.2021   Bulletin 2021/15**

(73) Proprietor: **Trio Healthcare Limited
Skipton, North Yorkshire BD23 2AA (GB)**

(72) Inventors:
• **PEARCE, Lloyd
  Skipton, North Yorkshire BD23 2AA (GB)**
• **LEE, Stewart
  Skipton, North Yorkshire BD23 2AA (GB)**

(74) Representative: **Murgitroyd & Company
Murgitroyd House
165-169 Scotland Street
Glasgow G5 8PL (GB)**

(56) References cited:
**WO-A1-02/066087          WO-A1-2007/093186
WO-A1-2017/158340**

(52) Cooperative Patent Classification (CPC): (Cont.)

C-Sets
**A61L 24/0094, C08L 83/04;
C09J 183/04, C08L 83/00, C08L 83/00, C08K 5/56,
C08K 3/26**

**Description**

Field of invention

The present invention relates to an adhesive skin compatible composition and in particular, although not exclusively, to a skin adhesive component suitable for coupling an ostomy appliance to the peristomal skin region of an ostomate.

Background art

An ostomate having a colostomy, ileostomy or urostomy requires a collection receptacle, such as a bag, be secured around the stoma being an artificial opening exiting through a person's abdomen. Typically, the collection bag is secured to the peristomal skin by means of an adhesive disc that may be required to be changed several times daily. As frequent removal of the adhesive disc causes irritation and damage to the skin, ostomy couplings have been developed in an attempt to increase comfort and improve wellbeing. Ostomy coupling arrangements commonly referred to as 'two-piece' systems comprise a first coupling part fitted with a ostomy bag to receive stoma discharge and a second coupling part (releasably connectable with the first) provided on a base layer (or wafer) that may be adhered to the peristomal skin. Accordingly, the ostomy bag may be changed readily without having to detach the base layer from the skin. Many different types of coupling arrangements have been proposed to maximise seal strength and minimise undesirable discharge. Example two-piece ostomy bag systems are described in EP 0687166; EP 1959881; US 4,846,798; WO 93/18725 and EP 0334489.

WO 2017/158340 A1 discloses a skin compatible adhesive for ostomy devices comprising: a silicone polymer network derived from the addition curing of a first part including a vinyl functionalised siloxane polymer and a second part including a silicon hydride containing crosslinker, in the presence of a metal catalyst; and a superabsorbent particulate distributed within the polymer network configured to absorb moisture from the skin; wherein the superabsorbent particulate has an average particle size less than 150 μm.

However, as a person is required to wear an ostomy coupling continuously, both two-piece and one-piece coupling arrangements must be completely interchanged at regular periods due to moisture uptake when in contact with the skin that cause erosion and breakdown of the adhesive disc. As will be appreciated, degradation of the coupling wafer will compromise the sealing contact with the skin and hence is very undesirable. Conventionally, the adhesive discs of ostomy appliances comprise hydrocolloids that possess high absorption capacity and are generally benign when in contact with the skin for long periods. However, conventional discs are disadvantageous due to their erosion and breakdown with moisture uptake. Additionally, such systems fail to provide a correct balance of the demands for a secure seal to be maintained around the stoma during attachment whilst allowing the coupling to be detached readily without causing skin irritation. Given the regularity with which even two-piece arrangement require changing, the compromise between adhesion and release is not found via such conventional systems. Accordingly, what is required is a coupling component or arrangement contactable with the skin that facilitates patient security and comfort and maximises wear time.

Summary of the Invention

The present invention is directed to a skin compatible component as defined in claim 1 and to a method of manufacturing a skin compatible component as defined in claim 11.

It is an objective of the present invention to provide an adhesive skin contactable component and in particular, although not exclusively, an ostomy coupling component or arrangement configured to exhibit enhanced moisture management when mounted in position at the peristomal skin. It is a specific objective to provide a skin attachable component being in particular an ostomy appliance adhesive disc that is resistant to erosion and breakdown in response to moisture so as to extend patient wear time.

The objectives are achieved, by providing a biocompatible silicone based layer, disc or pad positionable in contact with the skin, peri-stomal skin and/or peristomal skin that comprises moisture management characteristics including moisture absorption and breathability. In particular the objectives are achieved via a silicone adhesive layer being a room temperature vulcanisation silicone (RTV silicone) formed from a catalytic, addition cured, two-part system.

The objectives are further achieved specifically via a synthetic silicone gel based adhesive layer formed from a polyorganosiloxane derived silicone polymer network comprising Si-O and Si-C bonds in which a moisture control particulate is distributed within a foamed or expanded Si network. The expanded or foamed Si matrix incorporating the moisture control particulate may include a foaming agent or a residual or derivative compound of a foaming agent used to blow or expand the Si matrix to create an open or closed cell structure. The blowing agent may be a solid, liquid or gas. In preferred embodiments, the blowing agent is a bicarbonate and optionally sodium or potassium bicarbonate incorporated into the pre-cured two-part formulation.

The present polyorganosiloxane derived foamed material incorporating the superabsorbent (moisture absorbing) particulate exhibits the desired flexibility and appropriate expanded and free internal volume to achieve the desired water vapour transmission rate (WVTR) from the skin and through the matrix and also to accommodate the superabsorbent particulate . Such moisture management, including breathability, provides an ostomy wafer or gasket that may be worn comfortably by an ostomate for significantly longer time periods than is currently available for conventional systems. The moisture control additive, in the form of particulates, is selected to achieve the desired substrate adhesion at the skin with low or very low risk of maceration. The expanded porous Si matrix is advantageous to achieve enhanced WVTR from the skin and also to allow swelling of the superabsorbent particulate without compromising a cohesive strength of the material. The synergistic behaviour of the foamed Si material and absorbent particulate provides the desired balance between moisture absorption into the silicone matrix adhesion, cohesive properties and MVTR rates. The present base layer is particularly adapted to provide optimised transmission of moisture and vapour through the main body and across the full surface area of contact between the wafer and the skin. The present hydrophilic silicone based wafer accordingly maintains its shape profile, does not erode in the presence of moisture and is highly gas, vapour and moisture permeable.

The term *'particulate'* used herein encompasses polymer species having a micron or submicron size suitable for dispersion within a larger polymer network, gel phase and in particular a silicone polymer network (adapted or suitable to accommodate a superabsorbent particulate (SAP)). The moisture control SAP may be a hydrocolloid including a naturally occurring semi-synthetic or synthetic hydrocolloid.

Naturally-occurring hydrocolloids suitable for use with the subject invention include polysaccharides and cellulosic materials. Example polysaccharide hydrocolloids may comprise plant extracts including gums including in particular xanthan gum or pectin. Example cellulosic materials may comprise cellulose; carboxymethyl cellulose; carboxymethyl β-glucan; cross-linked sodium carboxymethyl cellulose; sodium carboxymethyl cellulose; methylcellulose; hydroxyethylcellulose and hydroxypropyl cellulose.

The terms *'foamed structure'*, *'expanded Si matrix'* and similar, as used herein, encompasses a polymer network having the desired porosity and open or closed cell structure to provide an interconnected network incorporating superabsorbent particles that provides specific pathways for the through-flow of gas, vapour, liquid and moisture from a first surface in contact with or proximate to the skin and a second surface facing away from and distal to the skin. The expanded or foamed configuration is achieved by incorporating a specific foaming or blowing agent within the pre-cured material formulation according to some embodiments. The blowing agent may persist as an identifiable species within the resulting fully cured Si polymer matrix or the blowing agent may decompose, evaporate or react during curing such that is does not persist and be identifiable within the resulting Si based material. The Si matrix is formed as an expanded silicone polymer network comprising Si-O and Si-C bonds. According to some embodiments, the expanded foamed Si matrix may be achieved by introducing a gas phase material into the liquid or gel phase Si composition prior to and/or during curing.

It is preferred that a blowing agent be incorporated within the pre-cured formulation to liberate a gas during curing with the blowing agent decomposing such that derivative components or residual compounds are identifiable within the cured Si matrix.

Semi-synthetic hydrocolloids may comprise starch or cellulose, such as starchacrylonitrile graft copolymer; a starch polyacrylate salt, and sulfuric acid, vinyl sulfonate, methacrylic acid, vinyl alcohol, vinyl chloride copolymers; guar gums, esterified uronic acid containing polymers such as hyaluronates and alginates, hyaluronate polyvinyl alcohol blends; chitosans formed from partial or complete deacetylation of chitin and/or depolymerisation.

Synthetic hydrocolloids suitable for use with the subject invention may comprise polyvinyl pyrrolidone; carboxyvinyl polymers and polyethylene oxide polymers; polymers of methyl vinyl ether and maleic acid and derivatives; polyvinyl alcohol, high molecular weight polyethylene glycols and polypropylene glycols; or polyethylene oxides.

Preferably, the moisture control SAP comprises sodium polyacrylate. Such a particulate has been found to be compatible with the as-formed addition cured silicone matrix and to absorb moisture released form the skin so as to swell appropriately without destroying the cohesive strength of the matrix hence the skin layer covering. Additionally, a sodium polyacrylate SAP contributes to providing the desired WVTR across the adhesive layer and facilitates the desired wicking action. In particular preferred sodium polyacrylate particulates have excellent water absorption property of the order of 350g/g $H_2O$ and 55g/g 0.9% NaCl. Additionally, such particulates be may configured with a 'fine' particle size selected such that a surface area/volume ratio is optimised to maximise the rate of moisture absorption. By way of example, a spherical sodium polyacrylate SAP having a nominal radius of 20 $\mu$m has a surface area to volume ratio five times that of an alternative nonsodium polyacrylate particle of nominal radius of 100$\mu$m. Additionally, sodium polyacrylate comprises a pH 6-7 and therefore specific/additional neutralisation is not required.

According to a first aspect of the present invention there is provided a skin compatible component attachable to mammalian skin comprising: a silicone polymer network derived from the addition curing of a first part including a vinyl functionalised siloxane polymer and a second part including a silicon hydride containing crosslinker, in the presence of a metal catalyst; and a superabsorbent particulate distributed within the polymer network configured to absorb moisture from the skin; wherein the silicone polymer network comprises a foamed structure.

Optionally, the superabsorbent particulate has an average particle size less than 150 $\mu$m. Optionally, the superabsorbent particulate comprises an average particle size in the range 10 to 40 $\mu$m, 15 to 35 $\mu$m or 20 to 30 $\mu$m.

The present skin compatible component via the foamed polyorganosiloxane derived silicone polymer exhibits high porosity, elasticity and low shear strength. The elastic nature of the present silicone adhesive layer provides a coupling assembly configured to retain its shape when stretched below its break point when allowed to relax. The present silicone based components is formed as a foamed silicone gel adhesive composition that also provides a balance of elastic and viscoelastic properties so as to achieve good adhesion of medical appliances to the skin whilst being readily peelable or removable from the skin when desired. Due to the moisture absorption and the moisture vapour transmission rate across the expanded silicone layer, the subject invention provides a device, coupling or appliance attachable to the skin that maintains and promotes skin health. The selection of the particulate described herein and the expanded silicone polymer matrix act together to achieve the desired chemical, physical and mechanical properties so as to provide a skin compatible component that does not degrade, erode or breakdown with the uptake of moisture. Accordingly, a skin contactable coupling is provided with considerably longer skin wear times relative to conventional devices.

The vulcanised silicone polymer is obtained by reacting an alkenyl-substituted polydiorganosiloxane, preferably a polydimethylsiloxane having silicon-bonded vinyl, allyl or hexenyl groups, and an organosiloxane containing silicon-bonded hydrogen atom and a catalyst for the reaction of the SiH groups with the Si-alkenyl (SiVi) groups, such as a platinum metal or its compounds or its complexes thereof. The ratio of SiVi:SiH can be 10:1 to 1:10. Preferred ratio of SiVi:SiH is 1:1. Altering the ratio of the reacting silicones from 1:1 ratio can change the adhesive properties of the layer. If a firmer, lower tack gel is required, the SiH component is higher than SiVi, and if a softer layer with higher tack is required, the SiVi component may be higher than SiH. The silicone compositions may be cured at ambient temperatures, but curing times can be reduced by exposure to elevated temperatures, from about 40° C to about 150° C. Non-limiting examples of such silicone polymer precursors include Soft Skin Adhesives SSA MG-7-1010, SSA 7-9900, 7-9950 from Dow Corning Corporation, Silpuran® 2114, 2117, 2122, 2130, 2140, 2142 and combinations thereof, SilGel® 612 from Wacker Chemicals. Hydrophilic group containing silicones, according to the present disclosure, may contain polar groups such as acid, amido, amino, sulfonyl, carboxyl, phosphate, phosphonate, etc., on the polydimethylsiloxane backbone. These groups could be present in an ionic form.

The moisture control particulate having an average particle size of less than 150 $\mu$m provides a '*fine*' particle distribution within the polymer network. This is advantageous to provide the moisture management characteristic of the subject invention and in particular to achieve a capillary action moisture transport through the foamed silicone polymer layer such that moisture (water) is readily transported across the expanded silicone polymer layer. Accordingly, the present foamed silicone polymer via the desired particle size of the moisture control particulate is configured to absorb and release moisture when in contact with the skin as the skin compatible component is worn by a patient for example at the peristomal skin. As will be appreciated, the surface area to volume ratio is an important factor in determining the active surface area for absorption of moisture from the skin in that for any given shape of particle, the surface area to volume ratio is inversely proportional to particle size. The present particulate size when incorporated within a foamed polyorganosiloxane derived silicone matrix has been found to act synergistically to achieve the desired performance of the skin compatible component with regard to adhesion, release, absorption and moisture vapour transmission rate. This is achieved in part as the expanded network, formed from the vulcanisation of the two-part system with foaming, comprises the desired cross linkage density and open micro-structure that entraps the particulate whilst allowing the particulate to swell in use without destroying the cohesiveness of the silicone layer covering.

A skin compatible component according to the subject invention comprises an optimised water vapour transmission rate so as to provide a component configured to balance transepidermal water loss (TEWL) from the skin with the water vapour transmission rates of the skin compatible component (covering the skin). This is achieved in part by the foamed structure and the sodium polyacrylate SAP as detailed herein. According to one aspect, the water vapour transmission rate (WVTR) of the present component may be in a range 100 to 1000 g/m$^2$.24h; 200 to 1000 g/m$^2$.24h; 300 to 1000 g/m$^2$.24h, 400 to 1000g/m$^2$.24h 500 to 1000 g/m$^2$.24h, 600 to 1000 g/m$^2$.24h, 700 to 1000 g/m$^2$.24h, 300 to 900 g/m$^2$.24h or 400 to 1000 g/m$^2$.24h using an upright cup method with a layer thickness of 635 $\mu$m to 750 $\mu$m. Such water vapour transmission rates may be correlated with a layer thickness of the silicone adhesive component having a thickness of 250$\mu$m to 1,000 $\mu$m; 500 $\mu$m to 1,000 $\mu$m; or 635 $\mu$m to 1,000 $\mu$m.

The silicone foam network formed from the addition curing of a first part and a second part is advantageous to provide the desired physical and mechanical properties of the resulting cured component. In particular, via the choice of the first and second part components the degree of crosslinking may be controlled so as to provide the desired network micro-structure to appropriately entrap the superabsorbent moisture control SAP as immobilised particles within the network. Additionally, the two-part addition cured composition provides the desired viscoelastic properties, adhesive tack, adhesive peel, moisture absorption, cohesive strength and WVTR. As will be appreciated, at least some of these characteristics may be considered contrary with regard to the attachment to mammalian skin and the present component and method of manufacture provides a balance of these considerations so as to optimise the component for moisture management at the skin.

Accordingly, the present component exhibits enhanced wear times relative to conventional hydrocolloid based components whilst providing the desired adhesion (adhesive tack) and adhesive peel so as to avoid problems of skin maceration and '*stripping*' on release. Additionally, the cohesive strength of the component is optimised via the two-part addition curing adhesive so as to maintain the integrity of the adhesive skin covering in response to moisture absorption/swelling of the skin covering for extended continuous wear times in excess of 70 hours and up to or beyond 400 hours. In particular, the two-part addition cured component provides a desired WVTR that is configured specifically to complement the skin TEWL. In particular, the present two-part addition cured foam may be considered advantageous over conventional one-part pressure sensitive adhesives (PSAs) that do not comprise the same open or closed cell structure with the same crosslinking density and cohesive strength.

Preferably, the superabsorbent particulate has an average particle size less than 150 $\mu$m. Preferably, the superabsorbent particulate comprises an average particle size in the range 10 to 40 $\mu$m, 15 to 35 $\mu$m or 20 to 30 $\mu$m. Preferably, the superabsorbent particulate is distributed within the polymer network at a concentration in the range 5 to 45 wt%, 10 to 40 wt%, 15 to 35 wt% or 20 to 30 wt%. The particle size according to the subject invention is advantageous to achieve the desired rate and speed of moisture absorption from the skin. Additionally, the present foamed configuration provides the required rate of moisture transmission across the adhesive layer due to wicking. In particular, a volume of moisture absorbed from the skin is appropriately transported across the adhesive silicone foam layer (and away from the skin) to avoid skin maceration, undesirable moisture welling and hence lifting/peeling of the silicone layer in use. The particle size also provides homogeneity and uniform distribution of the superabsorbent particulate (SAP) within the silicone foam that enhances the cohesive strength. In one aspect, the superabsorbent SAP and preferably the sodium polyacrylate (SAP) may comprise an absorption of 350 g.g$^{-1}$ (deionised water) or 55 g.g$^{-1}$ (0.9 weight% by volume of saline solution).

Preferably, an organosilicone resin is included in the first or second part prior to addition curing. Preferably, the organosilicone resin is an MQ resin. The organosilicone resin and in particular the MQ resin is advantageous to provide the desired balance between adhesive tack and peel/release characteristics. Optionally, the MQ resin has at least one reactive group such as hydroxyl, alkoxy, hydride, or vinyl functionalities. The silicone resin may comprise a cage-like oligosiloxane with the general formula of $R_nSiX_mO_y$, where R is a non-reactive substituent, usually Me or Ph, and X is a functional group H, OH, vinyl, or OR. These groups are further condensed to enhance or contribute to the resulting crosslinked polysiloxane network. Non-limiting examples of commercially available MQ resins are MQ-RESIN POWDER 803 TF from Wacker Chemical Corporation; VQM-135, VQM-146, HQM-105, HQM-107, SQO-299, and SQD-255 from Gelest Inc., Prosil 9932, MQOH-7 from SiVance, LLC.

Preferably, a cohesive strengthening agent is including in the first part or the second part prior to addition curing. Optionally, the cohesive strengthening agent comprises any one or a combination of the set of: fumed silica, fumed alumina, colloidal silica, nanoclays, silicates, silane treated organic polymers, polymeric metal oxides, and non-polymeric metal oxides. Preferably, the cohesive strengthening agent comprises fumed silica. The strengthening agent contributes to the cohesive strength characteristics of the component and assists with maintaining integrity of the silicone adhesive layer in response to moisture absorption by the SAP. In particular, the cohesive strengthening agent is further advantageous to minimise and eliminate layer residue once released from the skin. The strengthening agent is further advantageous to facilitate distribution of the SAP within the matrix. Furthermore, the cohesive strengthening agent further improves the integrity and cohesive strength at the perimeter edge of the silicone layer so as to reduce '*edge bleed*'. Non-limiting examples of cohesive strengthening agents of the present disclosure include silica, which could be fumed or precipitated silica such as AEROSIL® and SIPERNAT® grades, respectively, from Evonik Industries. The silica powders could be hydrophilic or hydrophobic, such as AEROSIL® 300, AEROSIL® 255, AEROSIL® R 812, AEROSIL® R 812 S, SIPERNAT® 120, SIPERNAT® 218, etc. Other non-limiting examples of cohesive strengthening agents include fumed alumina, colloidal silica, nanoclays, silicates, silane treated organic polymers, polymeric metal oxides, non-polymeric metal oxides, and the like.

Optionally, the cured skin compatible component may comprise a foaming agent, with the foaming agent being a residue, derivative or chemical entity as a result of the presence of a foaming agent introduced into the pre-cured formulation for example within part A, part B or both part A and part B. Optionally, the foaming agent may comprise any one or a combination of the following set of: water; a bicarbonate ion; a bicarbonate salt; a metal bicarbonate; azodicarbonamide; isocyanate; pentane; isopentane; cyclopentane; a fluorocarbon; a hydrofluorocarbon; a chlorinated fluorocarbon; a hydrocarbon; carbon dioxide.

Optionally, the component may comprise a reaction product, reactants or derivatives of an organic acid and an organic base. Optionally, the foaming agent may comprise an organic acid being any one or a combination of: a carboxylic acid, a sulfonic acid, lactic acid, acetic acid, foaming acid, citric acid, acetic acid, tartaric acid, L-tartaric acid, oxalic acid uric acid, a compound comprising a thiol group, an enol group or a phenol group; and/or wherein the foaming agent comprises an organic base being any one or a combination of: anolcoxide, an amidines, an amine, a phosphine, a sulphate, ammonia, perodene, acetone.

Preferably, the foaming agent comprises sodium bicarbonate. Optionally, the foaming agent may comprise water in addition to a metal/salt bicarbonate. Optionally, the foaming agent may further comprise an organic acid and/or an organic base. Preferably, an organic acid and an organic base are present within the pre-cured formulation in addition to a bicarbonate salt such as sodium bicarbonate or potassium bicarbonate.

According to a second aspect of the present invention there is provided an ostomy coupling comprising: a moisture and gas permeable support layer; an ostomy appliance or ostomy appliance connection provided at a first surface of the support layer; and a skin compatible component as claimed herein attached to a second surface of the support layer.

Optionally, the polyurethane layer comprises a thickness in the range 0.02 to 0.08 mm. The polyurethane layer may comprise a moisture vapour transmission rate of 1200 g.m$^{-2}$.24h$^{-1}$ using an upright cup method; a tensile strength of 40 to 50 MPa and an elongation of 500%. Preferably, the support layer may be regarded as porous so as to allow transmission of moisture, water vapour and gas through the support layer and hence provide a fully '*breathable*' skin adhesive covering pad or disc.

Preferably, the silicone matrix layer is protected by a release liner configured for quick and convenient removal prior to mounting of the silicone layer in direct contact with the skin.

Optionally, the release liner may comprise a thermoformable material, a fluoropolymer treated film, LDPE, polyethylene terephthalate (PET) or a polycarbonate based material.

Optionally, the support layer comprises any one or a combination of the set of: a breathable silicone layer; a polyethylene block amide polymer; a polytetrafluoroethylene polymer; an acrylic latex polymer; or a polyolefin based layer. Preferably, the support layer comprises polyurethane.

Optionally, the ostomy appliance comprises a bag or pouch attached to the support layer directly or via an intermediate layer. Optionally, the intermediate layer may comprise polyethylene or may comprise any one or a combination of the set of: a polyester disc; a non-woven polyester; a non-woven polyethylene; a polypropylene disc; a non-woven polypropylene. Optionally, the intermediate layer may be a single or double sided adhesive annular ring capable of adhering to one or more components forming part of the assembly. Such a configuration would avoid a requirement to weld the intermediate layer to other components for example via RF or sonic welding.

Preferably, the ostomy appliance connection comprise a first part of a two-part bag or pouch connection assembly (as are known within the art) in which a second part of the connection assembly is mounted at a bag or pouch, the first part and the second part capable of releasable mating to detachably secure the bag or pouch to the coupling.

Optionally, the coupling comprises an opening extending through the support layer and the skin compatible component. Optionally, the ostomy appliance comprises a bag or pouch attached to the support layer directly or via an intermediate layer. Optionally, the intermediate layer comprises polyethylene. Optionally, the intermediate layer comprises any one or a combination of the set of: a polyester disc; a polyester gauze; a polyethylene gauze; a polypropylene disc; a polypropylene gauze. Such materials may be configured as single or doubled sided adhesive rings or pads capable of adhering to other components within the assembly.

Optionally, the ostomy appliance connection comprises a first part of a bag or pouch connection assembly in which a second part of the connection assembly is mounted at a bag or pouch, the first part and the second part capable of releasable mating to detachably secure the bag or pouch to the coupling.

To further enhance the moisture management and vapour transmission of the present coupling (and in turn avoid skin maceration and the like) the coupling may further comprise an additional skin contact layer positioned at the skin facing side of the skin compatible component. Preferably, the additional skin contact layer is a silicone based adhesive component extending over selected regions of a skin facing side or surface of the skin compatible component.

Preferably the additional skin contact layer is formed non-continuously over the skin compatible component so as to provide exposed areas of the skin compatible component that are devoid of the additional skin contact layer. Accordingly, with the material in contact with the skin, adhesion is provided firstly via the additional skin contact layer and secondly by the exposed surface area regions of the skin compatible component (not covered by the additional skin contact layer). Such a configuration improves the skin friendliness of the present material during use by enhancing the breathability of the silicone layer and allowing moisture to be readily removed from the skin without causing irritation.

Preferably, the additional skin contact layer is a silicone adhesive layer provided on the skin facing surface of the skin compatible component and intended to be positioned in contact with the skin to adhere to the skin, the additional skin contact layer being noncontinuous over the skin facing surface of the skin compatible component such that areas of said surface are not concealed by the additional skin contact layer, said areas capable of positioning directly adjacent and/or in contact with the skin.

Optionally, the additional skin contact layer comprises a two-part catalysed, silicone elastomer. Optionally, the additional skin contact layer may comprise a composite of a plurality of different silicones and/or silicone based materials.

Optionally, the additional skin contact layer comprises the same material as the skin compatible component. Optionally, the additional skin contact layer comprises the same material as the skin compatible component but without the superabsorbent particulate. That is, the additional skin contact layer may comprise a silicone polymer network derived from the addition curing of a first part including a vinyl functionalised siloxane polymer and a second part including a silicon hydride containing crosslinker, in the presence of a metal catalyst.

Optionally, the additional skin contact layer may be formed as lines or dots on the skin facing surface of the skin compatible component. In such a configuration, the skin compatible component may be regarded as a substrate. Where the additional skin contact layer is formed as individual dots, flecks or marks, the pattern created by these dots may be uniform across the skin facing surface of the substrate. Alternatively, the pattern may change over the substrate surface and the material may comprise different patterns at different regions over the substrate. Where the additional skin contact layer comprises lines or ridges extending over the substrate, these lines may extend in different directions where the spacing between the lines or ridges is the same or variable across the substrate surface. Optionally, the lines may create a square, rectangular or circular grid pattern. Optionally, the lines or ridges are distributed at the skin facing surface to create geometric shapes. Preferably, the additional skin contact layer is bonded to the substrate and takes the form of concentric circles extending around a central aperture extending through the substrate and/or the multilayer coupling.

According to a third aspect of the present invention there is provided a method of manufacturing a skin compatible component attachable to mammalian skin comprising: mixing a first part including a vinyl functionalized siloxane polymer with a second part including a silicon hydride (Si-H) containing crosslinker to form a mix; incorporating within the mix a superabsorbent particulate ; incorporating within the mix at least one foaming agent; curing the mix via a metal catalyst; wherein the resulting addition cured silicone polymer network is a foam and the superabsorbent particulate is distributed within the foam.

Preferably, the first part or the second part further comprise an organosilicone resin. Preferably, the organosilicone resin comprises an MQ resin. Preferably, the organosilicone resin is a silicic acid, trimethylsilylester with silanol functionality. Preferably, the organosilicone resin is included in the mix at 0.2 to 10 wt%, 1 to 9 wt%, 2 to 8 wt%; 3 to 7 wt% or 4 to 6 wt%.

Preferably, the first or second part further comprises a cohesive strengthening agent. Preferably, the cohesive strengthening agent comprises fumed silica. Preferably, the fumed silica comprises a bulk density of 0.4 to 0.8 g/mL and a Brunauer-Emmitt-Teller (BET) specific surface area of 200 to 320 mm$^2$/g, 210 to 310 mm$^2$/g, 230 to 300 mm$^2$/g or 230 to 290 mm$^2$/g. Preferably, the fumed silica is included within the mix at 0.2 to 2.0 wt%, 0.3 to 2.0 wt%, 0.5 to 1.5 wt% or 0.8 to 1.2 wt%.

Preferably, the superabsorbent particulate and preferably the sodium polyacrylate particulate comprises a particle size in a range 10 to 40 $\mu$m, 15 to 35 $\mu$m or 20 to 30 $\mu$m. Preferably, the superabsorbent particulate and preferably the sodium polyacrylate particulate is included within the mix at 5 to 45 wt%, 15 to 35 wt%, 20 to 30 wt% or 22 to 28 wt%.

Preferably, the vinyl functionalized siloxane polymer comprises a vinyl-terminated polydimethylsiloxane (PDMS). Preferably, the silicon hydride (Si-H) containing crosslinker comprises a hydride-terminated polydimethylsiloxane (PDMS).

Preferably, the vinyl-terminated polydimethylsiloxane (PDMS) comprises a first vinyl-terminated PDMS having a mass average of 10,000 to 20,000 and a second vinyl-terminated PDMS having a mass average of 70, 000 to 100,000. These mass average polymer distributions provide a resulting cured silicone matrix with the desired crosslinking density, porosity and cohesive strength to withstand the swelling of the layer during use and not to degrade which would otherwise result in failure of the covering.

Preferably, the vinyl functionalized siloxane polymer comprises a vinyl-terminated polydimethylsiloxane (PDMS) and the silicon hydride (Si-H) containing crosslinker comprises a hydride-terminated polydimethylsiloxane (PDMS).

Preferably, the first part is included within the mix at 30 to 40 wt% or 31 to 35 wt% and the second part is included within the mix at 30 to 40 wt% or 33 to 37 wt%.

Preferably, the superabsorbent particulate is included within the mix at 20 to 30 wt% or 22 to 28 wt%. Preferably, an MQ resin included in the mix at 2 to 8 wt% or 3 to 7 wt%. Preferably, fumed silica included within the mix at 0.2 to 2.0 wt%, 0.5 to 1.5 wt% or 0.8 to 1.2 wt%.

Preferably, the first part further comprises an organoplatinum catalyst and a silicone-vinyl containing inhibitor and the second part further comprises a vinyl-terminated polydimethylsiloxane (PDMS) and preferrably the superabsorbant particulate is sodium polyacrylate. Optionally, the foaming agent may be included within the mix at 1 to 50 wt%; 1 to 40 wt%; 1 to 30 wt% or 1 to 20 wt%. Optionally, the foaming agent may comprise a single chemical species/compound or a combination of compounds where the wt% represents the combined concentration of the foaming agent.

Optionally, the foaming agent comprises sodium bicarbonate included within the mix in a range 1 to 10 wt%; 2 to 10 wt%; 3 to 10 wt%; 4 to 10 wt% or 5 to 10 wt%. Optionally, the sodium bicarbonate is included within the mix in a range 10 to 25 wt% or 10 to 15 wt%.

Optionally, the method may comprise adding water to the mix at 0.5 to 10 wt%, 0.5 to 5 wt% or 2 to 10 wt%.

According to a fourth aspect of the present invention there is provided a skin compatible component attachable to mammalian skin manufactured by the method as claimed herein.

According to a further aspect of the present invention there is provided a method of manufacturing an ostomy coupling comprising: applying a polyorganosiloxane derived silicone polymer mix to a moisture and gas permeable support layer; curing the organosiloxane derived silicone polymer at the support layer; attaching an ostomy appliance or ostomy appliance connection to a part of the support layer.

Brief description of drawings

A specific implementation of the present invention will now be described, by way of example only, and with reference to the accompanying drawings in which:

Figure 1 is a plan view of an ostomy appliance coupling according to a specific implementation of the present invention;

Figure 2 is a cross sectional view through A-A of the ostomy coupling of figure 1;

Figure 3 is a plan view of an ostomy appliance coupling according to a further specific implementation of the present invention;

Figure 4 is a cross sectional view through B-B of the ostomy coupling of figure 3;

Figure 5A is a cross section through an ostomy coupling of the type of figure 1 and 2 according to a further embodiment having an additional adhesive layer at the skin contact face of the coupling that is discontinuous over the skin contact face;

Figure 5B is a cross section through an ostomy coupling of the type of figure 3 and 4 according to a further embodiment having an additional adhesive layer at the skin contact face of the coupling that is discontinuous over the skin contact face; and

Figure 6 is a magnified image of a foamed structure of a silicone material as described and claimed herein.

Detailed description of preferred embodiment of the invention

A silicone polymer based skin compatible component according to the subject invention is particularly adapted for placement on mammalian skin to have a desired adhesion characteristic so as to remain in secure attachment to the skin (as the skin moves) when worn by a person whilst having the desired release characteristics to allow the component to be removed from the skin. The silicone based component is accordingly a hydrophilic humectant configured to absorb moisture into the silicone matrix without detriment to adhesion, cohesive properties and peel characteristics. The foamed component/material enables transmission of moisture vapour through the body of the matrix so as to allow the skin (in contact with the component) to breathe. Accordingly, the present silicone wafer, due in part, to the composition of the expanded/foamed silicone matrix is advantageous to balance moisture absorption with moisture and water vapour transmission to avoid skin maceration.

The subject invention is particularly suitable to secure medical appliances or devices to mammalian skin and in particular peri-stomal skin and peristomal skin. Such devices may include but are not limited to catheters, intravenous feeding lines, securement devices, wound dressings, therapeutic devices, drug delivery devices, ostomy appliances and the like.

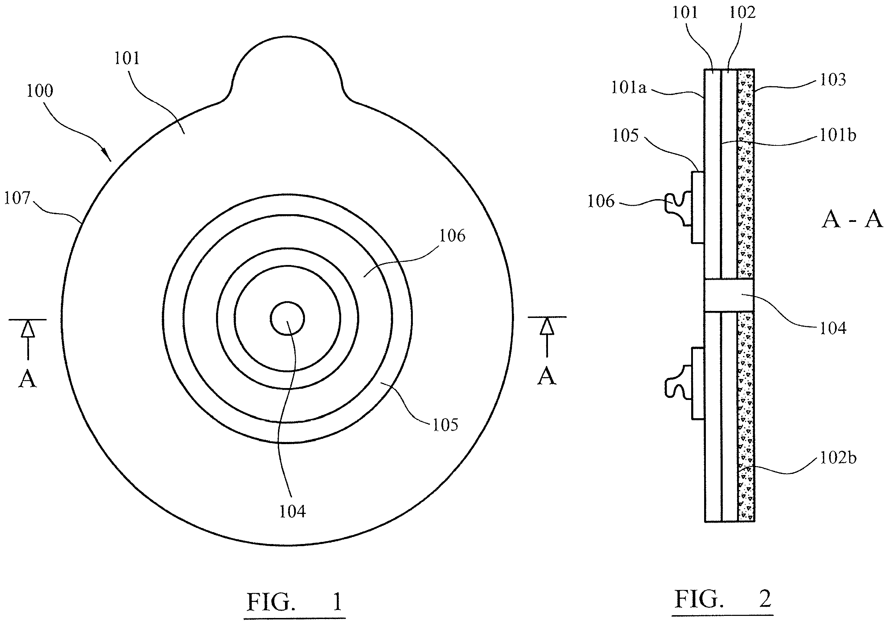

The subject invention will now be described with reference to a specific implementation in which the moisture absorbing particulate silicone based matrix forms a component part of an ostomy appliance coupling referred to as a 'base plate" of a 'two-piece' system. However, the subject invention may be utilised within a 'one-piece' ostomy appliance as will be appreciated. Referring to figures 1 and 2, a coupling assembly 100 comprises a moisture and water vapour permeable 'breathable' substrate layer 101 having a first surface 101a and a second surface 101b. According to the specific implementation, layer 101 comprises a polyurethane having a moisture vapour transmission rate (MVTR) of greater than 700 g.m$^{-2}$.24h$^{-1}$ and preferably a MVTR of 700 to 950 g.m$^{-2}$.24h$^{-1}$ using an upright cup method. According to the specific implementation, the polyurethane layer has an MVTR of 875 g.m$^{-2}$.24h$^{-1}$ using an upright cup method. A polyethylene disc 105 is secured to substrate first layer 101a via RF or ultrasonic welding or using an adhesive. The polyethylene disc 105 provides a mount for a first part 106 of an ostomy appliance coupling mechanism to releasably engage with a second part of the coupling mechanism provided at an ostomy appliance, in particular an ostomy bag. Coupling first part 106 is preferably formed as an annular flange capable of frictionally integrating and releasably locking with the second part of the coupling mechanism so as to provide a sealed coupling between an ostomy bag (not shown) and the coupling arrangement 100 of figures 1 and 2. According to the specific implementation, the first part 106 of the coupling mechanism (being any form of connection as will be appreciated and recognised by those skilled in the art) is secured to layer 105 via RF or ultrasonic welding. However, according to further implementations layer 105 may be a double sided adhesive tape (annular ring) suitable to bond to surface 101a and component part 106.

A silicone polymer foam layer 102 is applied to substrate second surface 101b by coating second surface 101b with a homogenous liquid phase non-cured silicone polymer mix that is then cured (i.e., room temperature vulcanised) in position at substrate 101. The silicone polymer layer 102 is coated and protected by a release liner 103. Release liner 103 according to the specific implementation comprises a fluoropolymer treated film. Liner 103 is releasably positioned over the silicone layer 102 and is removed prior to mounting of the coupling assembly 100 onto the skin of a person via mating contact with the silicone polymer layer surface 102b.

Layers 101, 102 are annular having a generally circular or oval disc shape profile. A through bore 104 extends through layers 101, 102 and is dimensioned to comprise an internal diameter slightly greater than an external diameter of a stoma with layers 101 and 102 having a generally circular outer perimeter 107 so that the present coupling may be regarded as a generally annular disc. Accordingly, coupling assembly 100 is configured for mounting in close fitting and sealing contact with the peristomal skin as is conventional with both one-piece and two-piece stoma appliances.

According to the specific implementation, polyurethane substrate 101 comprises a layer thickness of 20 $\mu$m to 50 $\mu$m and the silicone polymer layer 102 comprises a thickness of approximately 400 to 900 $\mu$m. Polyethylene disc105 comprises a thickness of 80 to 150 $\mu$m and release liner 103 comprises a thickness in the range 40 to 150 $\mu$m.

Figures 2 to 4 illustrate an ostomy appliance coupling according to a second embodiment being a variation of the embodiment described with reference to figures 1 to 2. According to the further embodiment, the breathable polyurethane layer 101, the silicone polymer foam layer 102 and the release liner 103 are as described for the first embodiment. However, in place of the polyethylene disc 105 a weldable non-woven layer 200 is secured to polyurethane first surface 101a. Non-woven layer 200 is also annular and comprises internal and external diameters corresponding to layers 101, 102 so as to form a welded extension of layers 101, 102 in the plane B-B. According to the specific implementation, a thickness of the non-woven layer 200 is 30 to 600 $\mu$m. The first part 106 of the appliance coupling mechanism is then welded to non-woven layer 200 via RF or ultrasonic welding.

A specific embodiment of the polymer layer 102 will now be described by reference to the following examples.

Polymer layer 102 is formed as a foamed silicone polymer matrix derived from the addition curing of a first part and a second part using a foaming/blowing agent. Supplementary components are included within the first and/or second parts to achieve the desired physical and mechanical characteristics of the resulting silicone network in addition to achieving the desired balance of viscoelastic properties, adhesive tack, adhesive peel, moisture absorption, cohesive strength and water vapour transmission rate (WVTR).

The two-part components may be cured/vulcanised at ambient temperatures (or elevated temperatures including in the range 30° to 150°). Curing/vulcanisation times may vary depending upon relative concentrations and components within the first and second parts. The base starting materials to create the resulting silicone network are detailed in table 1.

*Examples*

*Table 1 - starting materials of liquid phase non-cured mix*

| Component | Concentration %w/w | Purpose | Supplier |
|---|---|---|---|
| Silicone Silpuran® 2122 part A | 31-35 | Part A silicone + catalyst | Wacker Chemie |
| Silicone Silpuran® 2122 part B | 33-37 | Part B silicone crosslinker | Wacker Chemie |
| Aquakeep™ Sodium Polyacrylate | 23-27 | Moisture control, moisture transmission through silicone adhesive network | Sumitomo Seika Chemicals Co., Ltd |
| MQ Silanol Resin | 3-7 | Tackifier | Milliken™ SiVance LLC |
| Aerosil™ (Fumed silica) | 0.5-1.5 | Cohesive strengthener | Evonik Industries AG |
| Foaming agent | 0.5-25 | Foaming or blowing silicone matrix | - |

Example formulations were prepared using different foaming agents and different combination of foaming agents forming part of the starting materials identified in table 1. It is preferred that the foaming agent is included in the part B silicone composition together with the MQ Silanol Resin and the Sodium Polyacrylate. Foaming agents tested include sodium bicarbonate; sodium bicarbonate and water; potassium bicarbonate; potassium bicarbonate and water; water; sodium bicarbonate, acid and water. The acid may comprise citric, acetic or 1-tartaric acid.

*Sample preparations*

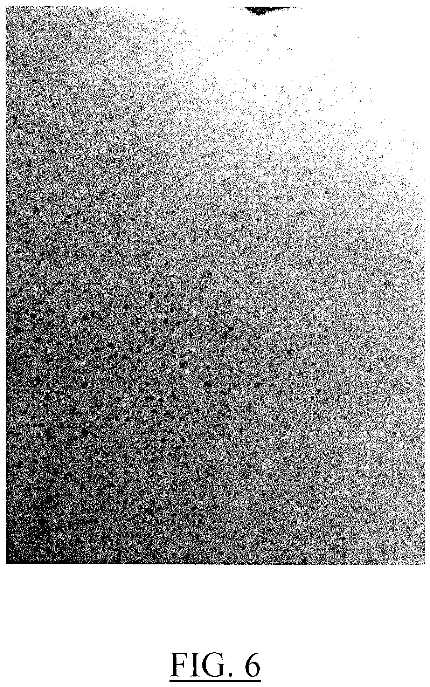

The Silpuran® based parts A and B were weighed and the other components, of the examples added at their respective concentrations. The components were mixed thoroughly to ensure complete dispersal of the components within the mix. In particular, thorough mixing reduces the risk of the SAPs agglomerating which would be detrimental to the moisture management characteristics across the full surface area of the skin compatible component. The above components were mixed using a medium to low shear mixing technique either by centrifusion or dispersal at 1000 to 3000 rpm. Surplus heat energy was removed by active cooling. Vacuum phase mixing was used as a final stage to provide an expanded/expanding liquid phase non-cured silicone formulation that was subsequently cured to solidify the foamed structure. A magnified image of the resulting foamed structure is shown in figure 6.

The laminate assembly 100 of figures 1 to 4 was manufactured by layering the foamed liquid phase silicone formulation onto the polyurethane layer surface 101b followed by room temperate vulcanisation (RTV) under controlled conditions. The release liner 103, the polyethylene disc 105 and the coupling first part 106 was then attached to form the multi component assembly 100.

The mix of table 1 was prepared incorporating each of the various foaming agents as detailed in table 2 and 3. The silicone base mix was then coated on a 20 micron polyurethane film at 1000 $\mu$m thickness. The assembly was then cured at 120 °C for 10 minutes. Moisture vapour transmission rates were then measured in accordance with standard SATM E96/E96M using two methods, method 1 - up-right cup and method 2 - inverted cup.

*Method 1*

    1. The coated test material was positioned on a hard/flat surface with the adhesive side facing upward.
    2. The Si gasket was weighed.
    3. A careful cut was made around the Si gasket using this as a template to size.
    4. The EZ cup was filled with ~100ml of distilled water.
    5. The test material was assembled into the cup with the adhesive surface facing into and across the cup.
    6. A PTFE spacer was assembled at a second Si gasket.
    7. The EZ cup lid was screwed down, to be securely seated, but not so tight as to wrinkle the test material.
    8. The assembled cup and distilled water was weighed.
    9. The assembled cup was transferred horizontally onto an incubator at 37°C (body temperature).
    10. The 'In' time and temperature was recorded.
    11. The cup left in the incubator for 24h.
    12. After 24 hours the cup was removed and the 'Out' time and temperature recorded.
    13. The cup was re-weigh immediately.
    14. The cup unit was dismantled and the upper two gaskets removed. The test material and Si gasket was reweighed.

*Method 2*

    1. Steps 1 through 8 were followed under method 1.
    2. The cup was transferred to the incubator and placed on the horizontal surface with the cup inverted so that the distilled water contacted the Si adhesive surface.
    3. Points 10 through 13 of method 1 were followed.
    4. The cup unit was dismantled and the upper two gaskets removed. Any excess water/droplets were carefully removed from the test material and the test material and Si gasket reweighed.

*Calculation and reporting*

    1. WVTR calculation

a. Calculate the weight loss from the cup (G) in the 24h period

b. Calculate the WVTR using the following;

i.

$$G/0.003166 = g.m^{-2}.24h^{-1}$$

2. Absorption calculation

a. Calculate the initial and separately the final weight of the test material by subtracting weight of Si gasket.

b. Calculate the weight increase of the test material and express the weight change as a percentage using;

i.

$$G \text{ (weight - gain)} / G \text{ (weight – initial)} \times 100 = \%w/w$$

The results of the WVTR assessment are shown in table 2.

*Table 2 -MVTR values for silcone matrix test materials incorporating various foaming agents*

| Foaming Agent | WVTR (g.m$^{-2}$. 24h$^{-1}$) | Vacuum | SUBSTRATE | NaHCO$_3$ or KHCO$_3$ w/w% | H$_2$O w/w% | 3N Citric acid w/w% | Temp °C | PULL |
|---|---|---|---|---|---|---|---|---|
| No foaming agent | 114 | Yes - Speed mix | 20μm PU | 0.0 | 0.00 | 0.00 | 100 | 1,000 |
| NaHCO$_3$ | 520 | No- Speed mix | 20μm PU | 16.7 | 0.00 | 0.00 | 100 | 1,000 |
| NaHCO$_3$ | 461 | No- Speed mix | 20μm PU | 1.0 | 0.00 | 0.00 | 150 | 1,000 |
| NaHCO$_3$ | 401 | No- Speed mix | 20μm PU | 14.3 | 0.00 | 0.00 | 100 | 1,000 |
| NaHCO$_3$ | 397 | No- Speed mix | 20μm PU | 4.7 | 0.00 | 0.00 | 100 | 1,000 |
| NaHCO$_3$ | 355 | No- Speed mix | 20μm PU | 9.0 | 0.00 | 0.00 | 100 | 1,000 |
| NaHCO$_3$ | 237 | No- Speed mix | 20μm PU | 7.5 | 0.00 | 0.00 | 100 | 1,000 |
| KHCO$_3$ | 376 | No-Hand mix | 20μm PU | 1.0 | 0.00 | 0.00 | 125 | 1,000 |
| KHCO$_3$ | 373 | No- Speed mix | 20μm PU | 1.0 | 0.00 | 0.00 | 125 | 1,000 |
| NaHCO$_3$ + Water | 834 | No- Speed mix | 20μm PU | 15.4 | 7.70 | 0.00 | 100 | 1,000 |

(continued)

| Foaming Agent | WVTR (g.m$^{-2}$.24h$^{-1}$) | Vacuum | SUBSTRATE | NaHCO$_3$ or KHCO$_3$ w/w% | H$_2$O w/w% | 3N Citric acid w/w% | Temp °C | PULL |
|---|---|---|---|---|---|---|---|---|
| NaHCO$_3$ + Water | 714 | No-Speed mix | 20μm PU | 8.9 | 1.80 | 0.00 | 100 | 1,000 |
| NaHCO$_3$ + Water | 321 | No-Speed mix | 20μm PU | 10.0 | 1.00 | 0.00 | 100 | 1,000 |
| NaHCO$_3$ + Citric Acid + Water | 758 | No-Speed mix | 20μm PU | 1.5 | 2.00 | 0.00 | 125 | 1,000 |
| NaHCO$_3$ + Citric Acid + without Water | 328 | No-Speed mix | 20μm PU | 1.5 | 0.00 | 0.00 | 125 | 1,000 |

MVTR values for a selection of the foamed materials of table 2 and some further foamed materials are shown in table 3.

*Summary Results*

*Table 3 - MVTR values for silcone matrix test materials incorporating various foaming agents where A and B are acid-base compounds that comprise the citric acid and the bicarbonate respectively.*

| Foaming Agent | WVTR (g.m$^{-2}$.24h$^{-1}$) | Vacuum | SUBSTRATE | NaHCO$_3$ or KHCO$_3$ w/w% | H$_2$O w/w% | 3N Citric acid w/w% | Temp °C | PULL |
|---|---|---|---|---|---|---|---|---|
| TD NaHCO$_3$ + Water | 8.34 | No-Speed mix | M124B-20 | 15.4 | 7.70 | 0.0 | 100 | 1,000 |
| A/B + Water | 758 | No-Speed mix | M124B-20 | 1.5 | 2.00 | 0.0 | 125 | 1,000 |
| TD NaHCO$_3$ + Water | 714 | No-Speed mix | M124B-20 | 8.9 | 1.80 | 0.0 | 100 | 1,000 |
| A/B + Water | 600 | Yes-Speed mix | M124B-20 | 0.9 | 0.00 | 10.0 | 125 | 760 |
| TD NaHCO$_3$ | 520 | No-Speed mix | M124B-20 | 16.7 | 0.00 | 0.0 | 100 | 1,000 |

As will be noted, whilst the above examples include water as an additive to formulations incorporating a bicarbonate salt, the blowing agent may comprises water exclusively or compositionally as a majority component of the blowing/foaming agent within the material composition of table 1.

In further example embodiments, the blowing agent of the material composition of table 1 comprises any one or a combination of a metal a bicarbonate salt; an azodicarbonamide; isocyanate; a volatile alkane; pentane; isopentane; cyclopentane; a gas phase compound to blow or expand the Si matrix, a fluorocarbon; a hydrofluorocarbon; a chlorinated fluorocarbon; a hydrocarbon; carbon dioxide or air.

As detailed in table 2 and 3, the moisture vapour transmission rates of the present silicone matrix material increased significantly due to the presence of the foaming agent. The WVTR values indicate an increase porosity of the Si matrix. Based on the results, the combination of a bicarbonate salt and water appreciably increased the WVTR. It will be appreciated that bicarbonate salts liberate carbon dioxide under thermal decomposition at the curing temperature range. Additionally, the aqueous phase represented as a gel evaporates at the cure temperature range to provide the blowing effect. Additionally, an acid-base reaction is effective to liberate a gaseous phase to expand the Si matrix during curing. This may be achieved via incorporation of bicarbonate salts and organic acids into the silicone formulation. Curing temperature was also found to effect the extent of foaming as indicated above. Without being bound by theory, it is believed the increased temperature during curing enhances decomposition of the bicarbonate salt to increase gas liberation during setting of the silicone matrix that in turn enhances the expanded/blown structure. Concentrations of the foaming additives were selected to avoid increasing/decreasing pH levels that would be detrimental to skin compatibility of the silicone formulations. It is believed the addition of water enhances the reaction of the bicarbonate and/or acid to increase the WVTR.

The present silicone adhesive is advantageous to provide a 'soft' atraumatic release from the skin so as to reduce the potential for skin stripping/damage. The present invention provides a balance of wear performance characteristics for skin compatibility including in particular edge lift, adhesion during wear, adhesion on removal, moisture control, skin condition after wear, skin trauma on removal and skin residue on removal. The present silicone adhesive is advantageous so as to be capable of being worn continuously during low, modest and high physical activity levels and movement as a wearer engages in such physical activity. The present skin adhesive is further advantageous to satisfy other ergonomic factors such as comfort during wear and conformity to skin/body topography.

The present foamed silicone polymer matrix incorporates an open or closed foam cell structure comprising holes, channels, voids, cavities or internal bubbles providing a porous structure to increase the water vapour transmission rate across the silicone layer. The foaming agent was configured specifically to expand the silicone polymer network to create the blown open structure. It is believed the enhanced open structure allows the through flow of vapour or moisture to enhance the moisture management characteristics of the silicone material in addition to facilitating moisture absorption by the SAPs within the silicone matrix.

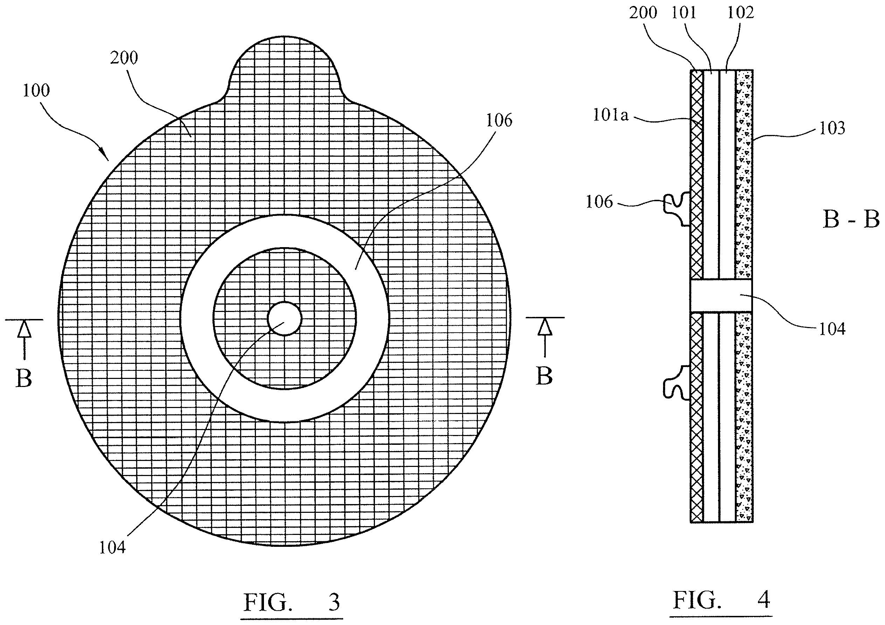

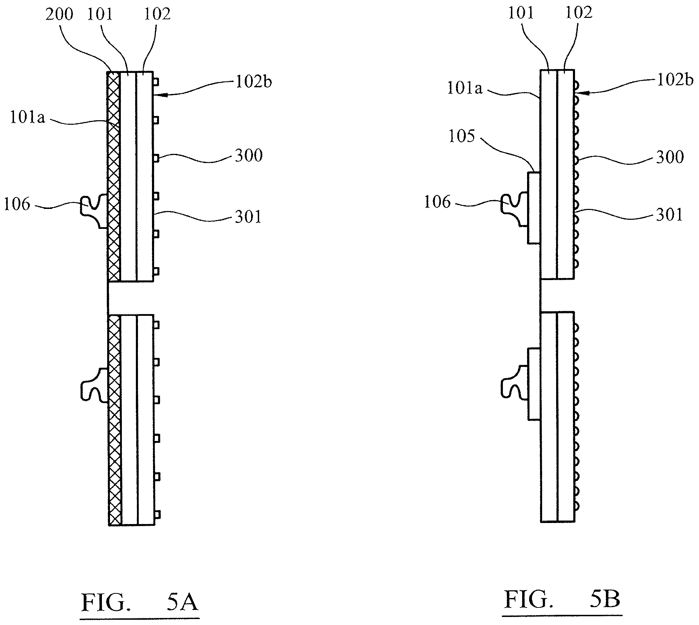

Further embodiments of the present invention are illustrated referring to figures 5A and 5B, with figure 5A being a variation of the embodiment of figure 1 and 2 and figures 5B being a variation of the embodiment of figure 3 and 4. According to both further embodiments, an additional skin contact layer 300 is adhered to and positioned at surface 102b of silicone polymer matrix layer 102. The additional skin contact layer 300 is preferably formed from the same material as layer 102. However, different materials may be used such as silicones or hydrocolloid based materials.

The additional skin contact layer 300 may be regarded as an additional adhesive layer formed from narrow ridges that are discontinuous over surface 102b such that additional skin contact layer 300 does not coat completely the surface 102b and there is provided regions 301 that are devoid of additional skin contact layer 300 with regions 301 being exposed surface areas of the silicone polymer matrix layer 102.

According to embodiments of figure 5A the pattern of the additional skin contact layer 300 at surface 102b is a rectangular grid pattern or concentric circles formed by uniform ridges extending across surface 102b. The spaces between the ridges may be equal in the respective directions across surface 102b.

The embodiment of Figure 5B comprises the additional skin contact layer 300 formed as a regular repeating array of nodes or bumps. The bumps may be separated from one another by a regular or uniform discreet separation distance such that the skin contact surface 102b of the silicone polymer matrix layer 102 is exposed at spacings 301 between the bumps 300.

According to a specific embodiment the additional skin contact layer 300 at surface 102b is formed as a series of concentric circles extending radially between central bore 104 and outer perimeter 107. The concentric circles (or other polygonal (i.e., rectangular) or nonpolygonal (i.e., oval) shapes) may be spaced apart from one another in the radial direction to be formed as discreet ridges separated by regions of exposed surface 102b. Such an embodiment is further beneficial to increase the strength and integrity of the moisture seal of the present coupling and reduce the risk of fluid leakage from under the coupling between the surface 102b and the skin.

**Claims**

**1.** A skin compatible component attachable to mammalian skin comprising:

a silicone polymer network derived from the addition curing of a first part including a vinyl functionalised siloxane polymer and a second part including a silicon hydride containing crosslinker, in the presence of a metal catalyst; and

a superabsorbent particulate distributed within the polymer network configured to absorb moisture from the skin; wherein the silicone polymer network comprises a foamed structure.

2. The component as claimed in claim 1 wherein the superabsorbent particulate has an average particle size less than 150 $\mu$m or an average particle size in the range 10 to 40 $\mu$m, 15 to 35 $\mu$m or 20 to 30 $\mu$m.

3. The component as claimed in any preceding claim wherein the superabsorbent particulate is distributed within the polymer network at a concentration in the range 5 to 45 wt%, 10 to 40 wt%, 15 to 35 wt% or 20 to 30 wt%.

4. The component as claimed in any preceding claim wherein the superabsorbent particulate comprises any one the set of:

- a naturally occurring hydrocolloid;
- a semi-synthetic hydrocolloid; or
- a synthetic hydrocolloid;
- a polysaccharide;
- a cellulose;
- hydroxyethylcellulose;
- carboxymethylcellulose;
- hydroxypropylcellulose;
- carboxymethyl $\beta$-glucan;
- cross-linked sodium carboxymethyl cellulose;
- sodium carboxymethyl cellulose;
- methylcellulose; or
- sodium polyacrylate.

5. The component as claimed in any preceding claim wherein an organosilicone resin and a cohesive strengthening agent are included in the first or second part prior to addition curing.

6. The component as claimed in claim 5 wherein the organosilicone resin is an MQ resin and the cohesive strengthening agent comprises any one or a combination of the set of: fumed silica, fumed alumina, colloidal silica, nanoclays, silicates, silane treated organic polymers, polymeric metal oxides, and non-polymeric metal oxides.

7. The component as claimed in any preceding claim comprising a foaming agent.

8. The component as claimed in any one of claims 1 to 6 further comprising a reaction product, reactants or derivatives of an organic acid and an organic base.

9. The component as claimed in any preceding claim wherein the foaming agent comprises:

water;
a bicarbonate ion;
a bicarbonate salt;
sodium bicarbonate;
a metal bicarbonate;
azodicarbonamide;
isocyanate;
pentane;
isopentane;
cyclopentane;
a fluorocarbon;
a hydrofluorocarbon;
a chlorinated fluorocarbon;
a hydrocarbon;
carbon dioxide;
an organic acid being any one or a combination of:

a carboxylic acid,

a sulfonic acid,
lactic acid,
acetic acid,
foaming acid,
citric acid,
tartaric acid,
L-tartaric acid,
oxalic acid
uric acid,

a compound comprising a thiol group, an enol group or a phenol group; or
an organic base being any one or a combination of:

anolcoxide,
an amidines,
an amine,
a phosphine,
a sulphate,
ammonia,
perodene,
acetone.

10. An ostomy coupling comprising:

a moisture and gas permeable support layer;
an ostomy appliance or ostomy appliance connection provided at a first surface of the support layer; and
a skin compatible component as claimed in any preceding claim attached to a second surface of the support layer.

11. A method of manufacturing a skin compatible component attachable to mammalian skin comprising:

mixing a first part including a vinyl functionalized siloxane polymer with a second part including a silicon hydride containing crosslinker to form a mix;
incorporating within the mix a superabsorbent particulate;
incorporating within the mix at least one foaming agent;
curing the mix via a metal catalyst;
wherein the resulting addition cured silicone polymer network is a foam and the superabsorbent particulate is distributed within the foam.

12. The method as claimed in claim 11 wherein:

• the superabsorbent particulate comprises sodium polyacrylate included within the mix at 5 to 45 wt%, 15 to 35 wt% or 20 to 30 wt%; and
• the first or second part further comprises an MQ resin and fumed silica.

13. The method as claimed in claims 11 or 12 further comprising a reaction product, reactants or derivatives of an organic acid and an organic base.

14. The method as claimed in claims 11 or 12 wherein the foaming agent comprises any one of the following set of:

water;
a bicarbonate ion;
a bicarbonate salt;
sodium bicarbonate;
a metal bicarbonate;
azodicarbonamide;
isocyanate;
pentane;
isopentane;

cyclopentane;
a fluorocarbon;
a hydrofluorocarbon;
a chlorinated fluorocarbon;
a hydrocarbon;
carbon dioxide;
an organic acid being any one or a combination of:

a carboxylic acid,
a sulfonic acid,
lactic acid,
acetic acid,
foaming acid,
citric acid,
tartarc acid,
L-tartaric acid,
oxalic acid,
uric acid,

a compound comprising a thiol group, an enol group or a phenol group; or
an organic base being any one or a combination of:

anolcoxide,
an amidines,
an amine,
a phosphine,
a sulphate,
ammonia,
perodene,
acetone.

15. The method as claimed in claim 14 wherein the foaming agent is included within mix at 1 to 30 wt%.


**Patentansprüche**

1.  Eine hautverträgliche Komponente, die auf Säugetierhaut angebracht werden kann und Folgendes beinhaltet:

    ein Siliconpolymernetz, das aus der Additionsvernetzung eines ersten Teils, der ein vinylfunktionalisiertes Siloxanpolymer umfasst, und eines zweiten Teils, der einen Siliciumhydrid enthaltenden Vernetzer umfasst, in Gegenwart eines Metallkatalysators, abgeleitet ist; und
    einen superabsorbierenden Schwebstoff, der innerhalb des Polymernetzes verteilt ist und konfiguriert ist, um Feuchtigkeit von der Haut zu absorbieren;
    wobei das Siliconpolymernetz eine geschäumte Struktur beinhaltet.

2.  Komponente gemäß Anspruch 1, wobei der superabsorbierende Schwebstoff eine durchschnittliche Partikelgröße von weniger als 150 µm oder eine durchschnittliche Partikelgröße im Bereich von 10 bis 40 µm, 15 bis 35 µm oder 20 bis 30 µm aufweist.

3.  Komponente gemäß einem der vorhergehenden Ansprüche, wobei der superabsorbierende Schwebstoff innerhalb des Polymernetzes in einer Konzentration im Bereich von 5 bis 45 Gew.-%, 10 bis 40 Gew.-%, 15 bis 35 Gew.-% oder 20 bis 30 Gew.-% verteilt ist.

4.  Komponente gemäß einem der vorhergehenden Ansprüche, wobei der superabsorbierende Schwebstoff ein Beliebiges aus dem folgenden Satz beinhaltet:

    • ein natürlich vorkommendes Hydrokolloid;
    • ein halbsynthetisches Hydrokolloid; oder

- ein synthetisches Hydrokolloid;
- ein Polysaccharid;
- eine Cellulose;
- Hydroxyethylcellulose;
- Carboxymethylcellulose;
- Hydroxypropylcellulose;
- Carboxymethyl-β-glucan;
- vernetzte Natrium-Carboxymethylcellulose;
- Natrium-Carboxymethylcellulose;
- Methylcellulose; oder
- Natriumpolyacrylat.

5. Komponente gemäß einem der vorhergehenden Ansprüche, wobei ein Organosiliconharz und ein Kohäsionsverstärker vor der Additionsvernetzung von dem ersten oder zweiten Teil umfasst werden.

6. Komponente gemäß Anspruch 5
wobei das Organosiliconharz ein MQ-Harz ist und der Kohäsionsverstärker ein Beliebiges oder eine Kombination aus dem folgenden Satz beinhaltet: pyrogener Kieselsäure, pyrogenem Aluminiumoxid, kolloidaler Kieselsäure, Nanotonen, Silicaten, mit Silan behandelten organischen Polymeren, polymeren Metalloxiden und nichtpolymeren Metalloxiden.

7. Komponente gemäß einem der vorhergehenden Ansprüche, die einen Schaumbildner beinhaltet.

8. Komponente gemäß einem der Ansprüche 1 bis 6, die ferner ein Reaktionsprodukt, Reaktanten oder Derivate einer organischen Säure und einer organischen Base beinhaltet.

9. Komponente gemäß einem der vorhergehenden Ansprüche, wobei der Schaumbildner Folgendes beinhaltet:

Wasser;
ein Bicarbonation;
ein Bicarbonatsalz;
Natriumbicarbonat;
ein Metallbicarbonat;
Azodicarbonamid;
Isocyanat;
Pentan;
Isopentan;
Cyclopentan;
einen Fluorkohlenwasserstoff;
einen Hydrofluorkohlenwasserstoff;
einen chlorierten Fluorkohlenwasserstoff;
einen Kohlenwasserstoff;
Kohlenstoffdioxid;
eine organische Säure, die eins oder eine Kombination von Folgendem ist:

einer Carbonsäure,
einer Sulfonsäure,
Milchsäure,
Essigsäure,
schäumender Säure,
Zitronensäure,
Weinsteinsäure,
L-Weinsteinsäure,
Oxalsäure
Harnsäure,

eine Verbindung, die eine Thiolgruppe, eine Enolgruppe oder eine Phenolgruppe beinhaltet; oder
eine organische Base, die eins oder eine Kombination von Folgendem ist:

Anolcoxid,
einem Amidin,
einem Amin,
einem Phosphin,
einem Sulfat,
Ammoniak,
Peroden,
Aceton.

10. Eine Stomakupplung, die Folgendes beinhaltet:

eine für Feuchtigkeit und Gas durchlässige Stützschicht;
eine Stomaeinrichtung oder Verbindung einer Stomaeinrichtung, die an einer ersten Oberfläche der Stützschicht bereitgestellt ist; und
eine hautverträgliche Komponente gemäß einem der vorhergehenden Ansprüche, die auf einer zweiten Oberfläche der Stützschicht angebracht ist.

11. Ein Verfahren zum Herstellen einer hautverträglichen Komponente, die auf Säugetierhaut angebracht werden kann und Folgendes beinhaltet:

Mischen eines ersten Teils, der ein vinylfunktionalisiertes Siloxanpolymer umfasst, mit einem zweiten Teil, der einen Siliciumhydrid enthaltenden Vernetzer umfasst, um eine Mischung zu bilden;
Inkorporieren eines superabsorbierenden Schwebstoffs in die Mischung;
Inkorporieren mindestens eines Schaumbildners in die Mischung;
Vernetzen der Mischung über einen Metallkatalysator;
wobei das resultierende additionsvernetzte Siliconpolymernetz ein Schaum ist und der superabsorbierende Schwebstoff innerhalb des Schaums verteilt ist.

12. Verfahren gemäß Anspruch 11, wobei:

• der superabsorbierende Schwebstoff Natriumpolyacrylat beinhaltet, das von der Mischung zu 5 bis 45 Gew.-%, 15 bis 35 Gew.-% oder 20 bis 30 Gew.-% umfasst wird; und
• der erste oder zweite Teil ferner ein MQ-Harz und pyrogene Kieselsäure beinhaltet.

13. Verfahren gemäß Anspruch 11 oder 12, das ferner ein Reaktionsprodukt, Reaktanten oder Derivate einer organischen Säure und einer organischen Base beinhaltet.

14. Verfahren gemäß Anspruch 11 oder 12, wobei der Schaumbildner ein Beliebiges aus dem folgenden Satz beinhaltet:

Wasser;
ein Bicarbonation;
ein Bicarbonatsalz;
Natriumbicarbonat;
ein Metallbicarbonat;
Azodicarbonamid;
Isocyanat;
Pentan;
Isopentan;
Cyclopentan;
einen Fluorkohlenwasserstoff;
einen Hydrofluorkohlenwasserstoff;
einen chlorierten Fluorkohlenwasserstoff;
einen Kohlenwasserstoff;
Kohlenstoffdioxid;
eine organische Säure, die eins oder eine Kombination von Folgendem ist:

einer Carbonsäure,
einer Sulfonsäure,

Milchsäure,
Essigsäure,
schäumender Säure,
Zitronensäure,
Weinsteinsäure,
L-Weinsteinsäure,
Oxalsäure,
Harnsäure,

eine Verbindung, die eine Thiolgruppe, eine Enolgruppe oder eine Phenolgruppe beinhaltet; oder
eine organische Base, die eins oder eine Kombination von Folgendem ist:

Anolcoxid,
einem Amidin,
einem Amin,
einem Phosphin,
einem Sulfat,
Ammoniak,
Peroden,
Aceton.

**15.** Verfahren gemäß Anspruch 14, wobei der Schaumbildner von der Mischung zu 1 bis 30 Gew.-% umfasst wird.

**Revendications**

**1.** Un composant compatible avec la peau pouvant être attaché à la peau d'un mammifère comprenant :

un réseau de polymère de silicone dérivé du durcissement par addition d'une première partie incluant un polymère de siloxane à fonctionnalité vinyle et d'une deuxième partie incluant un agent de réticulation contenant de l'hydrure de silicium, en présence d'un catalyseur métallique ; et
une matière particulaire superabsorbante répartie dans le réseau de polymère configurée pour absorber l'humidité provenant de la peau ;
où le réseau de polymère de silicone comprend une structure en mousse.

**2.** Le composant tel que revendiqué dans la revendication 1 où la matière particulaire superabsorbante a une taille de particule moyenne inférieure à 150 $\mu$m ou une taille de particule moyenne comprise dans l'intervalle de 10 à 40 $\mu$m, de 15 à 35 $\mu$m ou de 20 à 30 $\mu$m.

**3.** Le composant tel que revendiqué dans n'importe quelle revendication précédente où la matière particulaire superabsorbante est répartie dans le réseau de polymère à une concentration comprise dans l'intervalle de 5 à 45 % en poids, de 10 à 40 % en poids, de 15 à 35 % en poids ou de 20 à 30 % en poids.

**4.** Le composant tel que revendiqué dans n'importe quelle revendication précédente où la matière particulaire superabsorbante comprend n'importe quel ensemble parmi :

• un hydrocolloïde naturellement présent ;
• un hydrocolloïde semi-synthétique ; ou
• un hydrocolloïde synthétique ;
• un polysaccharide ;
• une cellulose ;
• de l'hydroxyéthylcellulose ;
• de la carboxyméthylcellulose ;
• de l'hydroxypropylcellulose ;
• du carboxyméthyl-$\beta$-glucane ;
• de la carboxyméthylcellulose de sodium réticulée ;
• de la carboxyméthylcellulose de sodium ;
• de la méthylcellulose ; ou

- du polyacrylate de sodium.

**5.** Le composant tel que revendiqué dans n'importe quelle revendication précédente où une résine d'organosilicone et un agent de renforcement cohésif sont inclus dans la première ou deuxième partie préalablement à un durcissement par addition.

**6.** Le composant tel que revendiqué dans la revendication 5
où la résine d'organosilicone est une résine MQ et l'agent de renforcement cohésif comprend n'importe quel ensemble ou une combinaison d'ensembles parmi : de la silice pyrogénée, de l'alumine pyrogénée, de la silice colloïdale, des nanoargiles, des silicates, des polymères organiques traités au silane, des oxydes métalliques polymères, et des oxydes métalliques non polymères.

**7.** Le composant tel que revendiqué dans n'importe quelle revendication précédente comprenant un agent moussant.

**8.** Le composant tel que revendiqué dans n'importe laquelle des revendications 1 à 6 comprenant en outre un produit de réaction, des réactifs ou des dérivés d'un acide organique et d'une base organique.

**9.** Le composant tel que revendiqué dans n'importe quelle revendication précédente où l'agent moussant comprend :

de l'eau ;
un ion bicarbonate ;
un sel de bicarbonate ;
du bicarbonate de sodium ;
un bicarbonate de métal ;
de l'azodicarbonamide ;
de l'isocyanate ;
du pentane ;
de l'isopentane ;
du cyclopentane ;
un fluorocarbone ;
un hydrofluorocarbone ;
un fluorocarbone chloré ;
un hydrocarbure ;
du dioxyde de carbone ;
un acide organique étant n'importe quel élément parmi ou une combinaison d'éléments parmi :

un acide carboxylique,
un acide sulfonique,
de l'acide lactique,
de l'acide acétique,
de l'acide moussant,
de l'acide citrique,
de l'acide tartrique,
de l'acide L-tartrique,
de l'acide oxalique,
de l'acide urique,

un composé comprenant un groupe thiol, un groupe énol ou un groupe phénol ; ou
une base organique étant n'importe quel élément parmi ou une combinaison d'éléments parmi :

de l'anolcoxyde,
une amidine,
une amine,
une phosphine,
un sulfate,
de l'ammoniac,
du pérodène,
de l'acétone.

**10.** Un couplage de stomie comprenant :

une couche de support perméable à l'humidité et aux gaz ;
un dispositif de stomie ou un raccord de dispositif de stomie fourni au niveau d'une première surface de la couche de support ; et
un composant compatible avec la peau tel que revendiqué dans n'importe quelle revendication précédente attaché à une deuxième surface de la couche de support.

**11.** Un procédé de fabrication d'un composant compatible avec la peau pouvant être attaché à la peau d'un mammifère comprenant :

le fait de mélanger une première partie incluant un polymère de siloxane à fonctionnalité vinyle avec une deuxième partie incluant un agent de réticulation contenant de l'hydrure de silicium afin de former un mélange ;
le fait d'incorporer une matière particulaire superabsorbante au mélange ;
le fait d'incorporer au moins un agent moussant au mélange ;
le fait de faire durcir le mélange par l'intermédiaire d'un catalyseur métallique ;
où le réseau de polymère de silicone durci par addition résultant est une mousse et la matière particulaire superabsorbante est répartie dans la mousse.

**12.** Le procédé tel que revendiqué dans la revendication 11 où :

• la matière particulaire superabsorbante comprend du polyacrylate de sodium inclus dans le mélange à hauteur de 5 à 45 % en poids, de 15 à 35 % en poids ou de 20 à 30 % en poids ; et
• la première ou deuxième partie comprend en outre une résine MQ et de la silice pyrogénée.

**13.** Le procédé tel que revendiqué dans les revendications 11 ou 12 comprenant en outre un produit de réaction, des réactifs ou des dérivés d'un acide organique et d'une base organique.

**14.** Le procédé tel que revendiqué dans les revendications 11 ou 12 où l'agent moussant comprend n'importe quel ensemble parmi les ensembles suivants :

de l'eau ;
un ion bicarbonate ;
un sel de bicarbonate ;
du bicarbonate de sodium ;
un bicarbonate de métal ;
de l'azodicarbonamide ;
de l'isocyanate ;
du pentane ;
de l'isopentane ;
du cyclopentane ;
un fluorocarbone ;
un hydrofluorocarbone ;
un fluorocarbone chloré ;
un hydrocarbure ;
du dioxyde de carbone ;
un acide organique étant n'importe quel élément parmi ou une combinaison d'éléments parmi :

un acide carboxylique,
un acide sulfonique,
de l'acide lactique,
de l'acide acétique,
de l'acide moussant,
de l'acide citrique,
de l'acide tartrique,
de l'acide L-tartrique,
de l'acide oxalique,
de l'acide urique,

un composé comprenant un groupe thiol, un groupe énol ou un groupe phénol ; ou
une base organique étant n'importe quel élément parmi ou une combinaison d'éléments parmi :

de l'anolcoxyde,
une amidine,
une amine,
une phosphine,
un sulfate,
de l'ammoniac,
du pérodène,
de l'acétone.

15. Le procédé tel que revendiqué dans la revendication 14 où l'agent moussant est inclus dans le mélange à hauteur de 1 à 30 % en poids.

FIG. 1

A - A

FIG. 2

FIG. 3

B - B

FIG. 4

EP 3 801 655 B1

FIG.   5A

FIG.   5B

FIG. 6

**EP 3 801 655 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0687166 A
- EP 1959881 A
- US 4846798 A
- WO 9318725 A
- EP 0334489 A
- WO 2017158340 A1